# EUROPÄISCHE PATENTANMELDUNG

(11) **EP 1 785 136 A2**
(43) Veröffentlichungstag der Anmeldung: **16.05.2007**
(21) Anmeldenummer: 07103257.7
(22) Anmeldetag: 25.07.2003
(51) Int. Cl.: A61K 31/46, A61K 31/56, A61K 31/135, A61K 31/167, A61K 31/58, A61K 45/06, A61P 11/00

(54) **Inhalative Arzneimittel enthaltend ein Anticholinergikum in Kombination mit Corticosteroiden und Betamimetika**

(30) Priorität: 17.08.2002 DE 10237739
(62) Teilanmeldung aus: 03793643.2
(71) Anmelder: Boehringer Ingelheim Pharma GmbH & Co. KG, 55216 Ingelheim (DE)
(72) Erfinder: Maede, Christopher John Montague, 88437, Maselheim (DE); Pairet, Michel, 88400, Biberach (DE); Pieper, Michael P., 88400, Biberach (DE)
(74) Vertreter: Hammann, Heinz

(57) **Zusammenfassung**

Die vorliegende Erfindung betrifft neuartige Arzneimittelkompositionen auf der Basis eines neuen Anticholinergikums, Corticosteroiden und Betamimetika, Verfahren zu deren Herstellung sowie deren Verwendung bei der Therapie von Atemwegserkrankungen.

## Beschreibung

Die vorliegende Erfindung betrifft neuartige Arzneimittelkompositionen auf der Basis eines neuen Anticholinergikums, Corticosteroiden und Betamimetika, Verfahren zu deren Herstellung sowie deren Verwendung bei der Therapie von Atemwegserkrankungen.

### Beschreibung der Erfindung

Die vorliegende Erfindung betrifft neuartige Arzneimittelkompositionen für die Inhalation auf der Basis eines neuen Anticholirtergikums, Corticosteroiden und Betamimetika, Verfahren zu deren Herstellung sowie deren Verwendung bei der Therapie von Atemwegserkrankungen.

Überraschenderweise kann ein unerwartet vorteilhafter therapeutischer Effekt, insbesondere ein synergistischer Effekt bei der Behandlung von entzündlichen oder obstruktiven Atemwegserkrankungen gezeigt werden, wenn ein neues Anticholinergikum gemeinsam mit einem oder mehreren Corticosteroiden und gemeinsam mit ein oder mehreren Betamimetika zur Anwendung gelangen. Aufgrund dieses synergistischen Effekts sind die erfindungsgemäßen Arzneimittelkombinationen in geringerer Dosierung einsetzbar, als dies bei der sonst üblichen Monotherapie der Einzelverbindungen der Fall ist. Dadurch lassen sich unerwünschte Nebenwirkungen, wie sie beispielsweise bei der Applikation von Corticosteroiden und Betamimetika auftreten können, vermindern.

Die vorstehend genannten Effekte können sowohl bei gleichzeitiger Applikation innerhalb einer einzigen Wirkstoffformulierung als auch bei sukzessiver Applikation der drei Wirkstoffkomponenten in getrennten Formulierungen beobachtet werden. Erfindungsgemäß bevorzugt ist die gleichzeitige Applikation der Wirkstofifbestandteile in einer einzigen Formulierung. Die erfindungsgemäßen Arzneimittel werden erfindungsgemäß bevorzugt inhalativ appliziert.

Im Rahmen der vorliegenden Erfindung gelangen als Anticholinergikum die Salze der Formel **1** 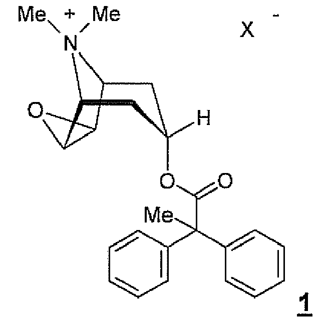 worin
X ⁻ ein einfach negativ geladenes Anion, vorzugsweise ein Anion ausgewählt aus der Gruppe bestehend aus Chlorid, Bromid, Iodid, Sulfat, Phosphat, Methansulfonat, Nitrat, Maleat, Acetat, Citrat, Fumarat, Tartrat, Oxalat, Succinat, Benzoat und p-Toluolsulfonat bedeutet zur Anwendung.

Bevorzugt gelangen die Salze der Formel **1** zur Anwendung, worin
X ⁻ ein einfach negativ geladenes Anion ausgewählt aus der Gruppe Chlorid, Bromid, 4-Toluolsulfonat und Methansulfonat, bevorzugt Bromid, bedeutet.

Besonders bevorzugt gelangen die Salze der Formel **1** zur Anwendung, worin
X ⁻ ein einfach negativ geladenes Anion ausgewählt aus der Gruppe Chlorid, Bromid und Methansulfonat, bevorzugt Bromid, bedeutet.

Erfindungsgemäß besonders bevorzugt ist dasjenige Salz der Formel **1**, in dem X ⁻ für Bromid steht.

Die Salze der Formel **1** sind aus der Internationalen Patentanmeldung WO 02/32899 bekannt. Eine Bezugnahme auf die Salze der Formel **1** schließt eine Bezugnahme auf deren gegebenenfalls erhältliche Hydrate und Solvate mit ein.

Im Rahmen der vorliegenden Patentanmeldung ist eine explizite Bezugnahme auf das pharmakologisch aktive Kation der Formel 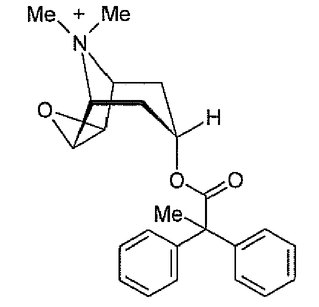 durch Verwendung der Bezeichnung **1'** zu erkennen. Eine Bezugnahme auf Verbindungen **1** schließt naturgemäß eine Bezugnahme auf das Kation **1'** mit ein.

Im Rahmen der vorliegenden Erfindung werden unter Corticosteroiden (im Folgenden **2**) Verbindungen verstanden, die ausgewählt sind aus der Gruppe bestehend aus Flunisolide, Beclomethasone, Triamcinolone, Budesonid, Fluticasone, Mometasone, Ciclesonide, Rofleponide, GW 215864, KSR 592, ST-126 und Dexametasone. Bevorzugt ist die Verbindung **2** ausgewählt aus der Gruppe bestehend aus Flunisolide, Beclomethasone, Triamcinolone, Budesonid, Fluticasone, Mometasone, Ciclesonide und Dexametasone. Besonders bevorzugt ist die Verbindung **2** ausgewählt aus Budesonid, Fluticasone, Mometasone und Ciclesonide. Gegebenfalls wid im Rahmen der vorliegenden Patentanmeldung statt der Bezeichnung Corticosteroide **2** auch nur die Bezeichnung Steroide **2** verwendet.

Eine Bezugnahme auf Steroide **2** schließt im Rahmen der vorliegenden Erfindung eine Bezugnahme auf Salze oder Derivate **2'**, die von den Steroiden gebildet werden können, mit ein. Als mögliche Salze oder Derivate **2'** werden beispielsweise genannt: Natriumsalze, Sulfobenzoate, Phosphate, Isonicotinate, Acetate, Propionate, Dihydrogenphosphate, Palmitate, Pivalate oder Furoate. Gegebenenfalls können die Verbindungen der Formel 2 auch in Form ihrer Hydrate vorliegen.

Als Betamimetika **3** kommen erfindungsgemäß Bambuterol, Bitolterol, Carbuterol, Clenbuterol, Fenoterof, Formoterol, Hexoprenalin, Ibuterol, Pirbuterol, Procaterol, Reproterol, Salmeterol, Sulfonterol, Terbutalin, Tolubuterol, 4-Hydroxy-7-[2-{[2-{[3-(2-phenylethoxy)propyl]sulfonyl}ethyl]-amino}ethyl]-2(3H)-benzothiazolon, 1-(2-Fluoro-4-hydroxyphenyl)-2-[4-(1-benzimidazolyl)-2-methyl-2-butylamino]ethanol, 1-[3-(4-Methoxybenzyl-amino)-4-hydroxyphenyl]-2-[4-(1-benzimidazolyl)-2-methyl-2-butylamino]ethanol, 1-[2H-5-Hydroxy-3-oxo-4H-1,4-benzoxazin-8-yl]-2-[3-(4-N,N-dimethylaminophenyl)-2-methyl-2-propylamino]ethanol, 1-[2H-5-Hydroxy-3-oxo-4H-1,4-benzoxazin-8-yl]-2-[3-(4-methoxyphenyl)-2-methyl-2-propylamino]ethanol, 1-[2 H-5-Hydroxy-8-oxa-4H-1,4-benzoxazin-8-yl]-2-[3-(4-n-butyloxyphenyl)-2-methyl-2-propylamino]ethanol 1-[2H-5-Hydroxy-3-oxo-4H-1,4-benzoxazin-8-yl]-2-{4-[3-(4-methoxyphenyl)-1,2,4-triazol-8-yl]-2-methyl-2-butylamino}ethanol, 5-Hydroxy-8-(1-hydroxy-2-isopropylaminobutyl)-2H-1,4-benzoxazin-3-(4H)-on, 1-(4-Amino-3-chloro-5-trifluormethylphenyl)-2-tert-butylamino)ethanol oder 1-(4-Ethoxycarbonylamino-3-cyano-5-fluorophenyl)-2-(tert-butylamino)ethanol in Betracht.

Erfindungsgemäß bevorzugt werden als Betamimetika **3** in der erfindungsgemäßen Wirkstoffkombination eingesetzt Formoterol, Salmeterol,
4-Hydroxy-7-[2-{[2-{[3-(2-phenylethoxy)propyl]sulfonyl}ethyl]-amino}ethyl]-2(3H)-benzothiazolan,
1-(2-Fluoro-4-hydroxyphenyl)-2-[4-(1-benzimidazolyl)-2-methyl-2-butylamino]ethanol ,
1-[3-(4-Methoxybenzyl-amino)-4-hydroxyphenyl]-2-[4-(1-benzimidazolyl)-2-methyl-2-butylamino]ethanol,
1-[2H-5-Hydroxy-3-oxo-4H-1,4-benzoxazin-8-yl]-2-[3-(4-N,N-dimethylaminophenyl)-2-methyl-2-propylamino]ethanol,
1-[2H-5-Hydroxy-3-oxo-4H-1,4-benzoxazin-8-yl]-2-[3-(4-methoxyphenyl)-2-methyl-2-propylamino]ethanol,
1-[2H-5-Hydroxy-3-oxo-4H-1,4-benzoxazin-8-yl]-2-[8-{4-n-butyloxyphenyl)-2-methyl-2-propylamino]ethanol oder
1-[2H-5-Hydroxy-3-oxo-4H-1,4-benzoxazin-8-yl]-2-{4-[3-(4-methoxyphenyl)-1,2,4-triazol-3-yl]-2-methyf-2-butylamino}ethanol.

Als Betamimetika **3** kommen erfindungsgemäß besonders bevorzugt Salmeterolsalze oder Formoterolsalze in Betracht. Eine Bezugnahme auf den Begriff Betamimetika **3** schließt eine Bezugnahme auf die jeweiligen Enantiomere oder deren Gemische mit ein. Beispielsweise schließt eine Bezugnahme auf die erfindunsgemäß besonders bevorzugten Verbindungen **3**, die Salze des Salmeterols und Formoterols, dementsprechend die jeweiligen enantiomeren Salze des R-Salmeterols, S-Salmeterols, *R,R*-Formoterols, *S,S*-Formoterols, *R,S*-Formoterofs, *S,R*-Formoterols sowie deren Gemische mit ein, wobei den enantiomeren Salzen des R-Salmeterols und *R,R*-Formoterols eine besondere Bedeutung zukommt. Die Verbindungen **3** können erfindungsgemäß ferner in Form ihrer Hydrate oder Solvate vorliegend.

Im Rahmen der vorliegenden Erfindung ist mit der Bezugnahme auf Verbindungen **3** eine Bezugnahme auf physiologisch verträgliche Säureadditionssalze zu verstehen. Als physiologisch verträgliche Säureadditionssalze der Betamimetika **3** werden erfindungsgemäß pharmazeutisch verträgliche Salze verstanden, die ausgewählt aus den Salzen der Salzsäure, Bromwasserstoffsäure, Schwefelsäure, Phosphorsäure, Methansulfionsäure, Essigsäure, Fumarsäure, Bernsteinsäure, Milchsäure, Zitronensäure, Weinsäure, 1-Hydroxy-2-naphthalincarbonsäure, Zimtsäure, 4-Phenylzimtsäure, Diflunisal oder Maleinsäure sind. Gegebenenfalls können zur Herstellung der Salze **3** auch Mischungen der vorgenannten Säuren eingesetzt werden.
Erfindungsgemäß bevorzugt sind die Salze der Betamimetika **3** ausgewählt aus der Gruppe bestehend aus Hydrochlorid, Hydrobromid, Sulfat, Phosphat, Fumarat Methansulfonat, 4-Phenylcinnamat, Diflunisal und Xinafoat. Besonders bevorzugt sind die Salze von **3** im Falle des Salmeterols ausgewählt aus denjenigen Salzen, die eine Löslichkeit in Wasser von von 0,1 mg/ml oder weniger, bevorzugt 0,05 mg/ml oder weniger, besonders bevorzugt 0,04 mg/ml oder weniger aufweisen. Indiesem Zusammenhang seien als besonders bevorzugte Salze des Almeterols das Xinafoat, das 4-Phenylcinnamat und das Diflunisal genannt. Besonders bevorzugte Salze **3** des Salmeterols weisen in Wasser eine Löslichkeit von 0,035 mg/ml oder weniger auf, wie beispielsweise das 4-Phenylcinnamat oder das Diflunisal. Besonders bevorzugt sind die Salze von **3** im Falle des Formoterols ausgewählt aus Hydrochlorid, Sulfat und Fumarat, von denen das Hydrochlorid und Fumarat besonders bevorzugt sind. Erfindungsgemäß von herausragender Bedeutung ist Formoterolfumarat.

Erfolgt im Rahmen der vorliegenden Erfindung eine Bezugnahme auf nicht in der Salzform vorliegende Betamimetika, so wird dies durch eine Bezugnahme auf Verbindungen **3'** erkennbar. Beispielsweise wird unter den erfindungsgemäß bevorzugten, nicht in der Salzform vorliegenden Betamimetika **3'** die freie Base des Formoterols oder des Salmeterols verstanden, wohingegen es sich bei den erfindungsgemäß besonders bevorzugten Verbindungen **3** beispielsweise um Salmeterolxinafoat, Salmeterol-4-phenylcinnamat oder Formoterolfumarat handelt.

Im Rahmen der vorliegenden Erfindung werden die Betamimetika **3** gegebenenfalls auch bezeichnet als Sympathomimetika oder Beta-2-Rezeptor-Agonisten (β2-Agonisten). All diese Bezeichnungen sind im Rahmen der vorliegenden Erfindung als äquivalent anzusehen.

Die Applikation der erfindungsgemäßen Arzneimittelkombinationen aus **1**, **2** und **3** erfolgt erfindungsgemäß auf inhalativem Wege. Hierbei können erfindungsgemäß bevorzugt geeignete Inhaltionspulver, die in geeignete Kapseln (Inhaletten) abgefüllt mittels entsprechender Pulverinhalatoren appliziert werden, zum Einsatz kommen.

Ein Aspekt der vorliegenden Erfindung betrifft dementsprechend ein Arzneimittel, welches eine Kombination aus **1**, **2** und **3** enthält.

Ein weiterer Aspekt der vorliegenden Erfindung betrifft ein Arzneimittel, welches ein oder mehrere Salze **1,** ein oder mehrere Verbindungen **2** und ein oder mehrere Verbindungen **3**, gegebenfalls in Form ihrer Solvate oder Hydrate enthält. Hierbei können die Wirkstoffe entweder gemeinsam in einer einzigen Darreichungsform, in zwei oder in drei getrennten Darreichungsformen enthalten sein. Erfindungsgemäß bevorzugt sind Arzneimittel, die die Wirkstoffe **1**, **2** und **3** in einer einzigen Darreichungsform enthalten.

Ein weiterer Aspekt der vorliegenden Erfindung betrifft ein Arzneimittel, welches neben therapeutisch wirksamen Mengen von **1**, **2** und **3** einen pharmazeutisch verträglichen Trägerstoff enthält. Ein weitere Aspekt der vorliegenden Erfindung betrifft ein Arzneimittel, welches neben therapeutisch wirksamen Mengen von **1**, **2** und **3** keinen pharmazeutisch verträglichen Trägerstoff enthält.

Die vorliegende Erfindung betrifft ferner die Verwendung von **1, 2** und **3** zur Herstellung eines therapeutisch wirksame Mengen von **1, 2** und **3** enthaltenden Arzneimittels zur Behandlung von entzündlichen und/oder obstruktiven Atemwegserkrankungen, insbesondere von Asthma und/oder chronisch obstruktiven Lungenerkrankungen (COPD) durch simultane oder sukzessive Applikation. Ferner können die erfindungsgemäßen Arzneimittelkombinationen zur Herstellung eines Arzneimittels zur Behandlung von beispielsweise cystischer Fibrose oder allergischer Alveolitis (Farmers Lung) durch simultane oder sukzessive Applikation Verwendung finden. Eine Anwendung der erfindungsgemäßen Wirkstoffkombinationen erfolgt nur dann nicht, wenn eine Behandlung mit einem der pharmazeutisch aktiven Wirkstoffe kontraindiziert ist.

Die vorliegende Erfindung zielt ferner auf die simultane oder sukzessive Verwendung therapeutisch wirksamer Dosen der Kombination vorstehender Arzneimittel **1**, **2** und **3** zur Behandlung von entzündlichen oder obstruktiven Atemwegserkrankungen, insbesondere von Asthma und/oder chronisch obstruktiven Lungenerkrankungen (COPD), sofern eine Behandlung mit Steroiden oder Betamimetika aus therapeutischer Sicht nicht kontraindiziert ist, durch simultane oder sukzessive Applikation. Die vorliegende Erfindung zielt ferner auf die simultane oder sukzessive Verwendung therapeutisch wirksamer Dosen der Kombination vorstehender Arzneimittel **1, 2** und **3** zur Behandlung von beispielsweise cystischer Fibrose oder allergischer Alveolitis (Farmers Lung).

In den erfindungsgemäßen Wirkstoffkombinationen aus **1**, **2** und **3** können die Bestandteile **1**, **2** und **3** in Form ihrer Enantiomere, Gemische der Enantiomere oder in Form der Racemate enthalten sein. Beispielsweise enthalten die erfindungsgemäßen Arzneimittel die Wirkstoffe **1**, **2** und **3** in einer solchen Menge, daß eine einmalige Applikation einer Dosierung der Wirkstoffkombination **1**, **2** und **3** von 1 bis 10000µg, bevorzugt von 10 bis 2000µg entspricht.
Die Verhältnisse, in denen die Wirkstoffe **1**, **2** und **3** in die erfindungsgemäßen Wirkstoffkombinationen eingesetzt werden können, sind variabel. Die Wirkstoffe **1**, **2** und **3** können gegebenfalls in Form ihrer Solvate oder Hydrate vorliegen. Je nach Wahl der Verbindungen **1**, **2** und **3** variieren die im Rahmen der vorliegenden Erfindung einsetzbaren Gewichtsverhältnisse aufgrund des unterschiedlichen Molekulargewichts der verschiedenen Verbindungen sowie aufgrund ihrer unterschiedlichen Wirkstärke. In der Regel können die erfindungsgemäßen Arzneimittelkombinationen die Verbindungen **1'** und **2** in Gewichtsverhältnissen enthalten, die in einem Bereich von 1:250 bis 250:1, bevorzugt von 1:150 bis 150:1, liegen. Bei den besonders bevorzugten Arzneimittelkompositionen, die neben **1**' eine Verbindung ausgewählt aus der Gruppe bestehend aus Budesonid, Fluticasone, Mometasone, ST-126 und Ciclesonide als Steroid **2** enthalten, liegen die Gewichtsverhältnisse von **1'** zu **2** besonders bevorzugt in einem Bereich von etwa 1:40 bis 40:1, ferner bevorzugt von 1:30 bis 30:1.

Beispielsweise und ohne den Umfang der Erfindung darauf zu beschränken, können bevorzugte erfindungsgemäße Kombinationen aus **1** und **2** das Kation **1'** und eines der vorstehend genannten bevorzugt zur Anwendung gelangenden Steroide **2** in den folgenden Gewichtsverhältnissen enthalten: 1:20; 1:19; 1:18; 1:17; 1:16; 1:15; 1:14; 1:13; 1:12; 1:11; 1:10; 1:9; 1:8; 1:7; 1:6; 1:5; 1:4; 1:3; 1:2; 1:1; 2:1; 3:1; 4:1; 5:1; 6:1; 7:1; 8:1; 9:1; 10:1; 11:1; 12:1; 13:1; 14:1; 15:1; 16:1; 17:1; 18:1; 19:1; 20:1.

Die Anwendung der erfindungsgemäßen Arzneimittel enthaltend die Kombinationen aus **1** und **2** erfolgt üblicherweise so, dass **1'** und **2** gemeinsam in Dosierungen von 5 bis 500µpbevorzugt von 10 bis 2000µg, besonders bevorzugt von 15 bis 1000µg, ferner bevorzugt von 20 bis 800µg, erfindungsgemäß bevorzugt von 30 bis 700µg, bevorzugt von 40 bis 600µg, bevorzugt von 50 bis 500µg, bevorzugt von 40 bis 500µg, besonders bevorzugt von 50 bis 400µg pro Einmalgabe enthalten sind. Beispielsweise enthalten erfindungsgemäße Kombinationen aus **1** und **2** eine solche Menge an **1'** und Steroid **2**, dass die Gesamtdosierung pro Einmalgabe etwa 35µg, 45µg, 50µg, 55µg, 60µg, 65µg, 70µg, 75µg, 80µg, 85µg, 90µg, 95µg, 100µg, 105µg, 110µg, 115µg, 120µg, 125µg, 130µg, 135µg,140 µg, 145µg, 150µg, 155µg, 160µg, 165µg, 170µg, 175µg, 180µg, 185µg, 190µg, 195µg, 200µg, 205µg, 210µg, 215µg, 220µg, 225µg, 230µg, 235µg, 240µg, 245µg, 250µg, 255µg, 260µg, 265µg, 270µg, 275µg, 280µg, 285µg, 290µg, 295µg, 300µg, 305µg, 310µg, 315µg, 320µg, 325µg, 330µg, 335µg, 340µg, 345µg, 350µg, 355µg, 360µg, 365µg, 370µg, 375µg, 380µg, 385µg, 390µg, 395µg, 400µg, 405µg, 410µg, 415µg, 420µg, 425µg, 430µg, 435µg, 440µg, 445µg, 450µg, 455µg, 460µg, 465µg, 470µg, 475µg, 480µg, 485µg, 490µg, 495µg, 500µg, 505µg, 510µg, 515µg, 520µg, 525µg, 530µg, 535µg, 540µg, 545µg, 550µg, 555µg, 560µg, 565µg, 570µg, 575µg, 580µg, 585µg, 590µg, 595µg, 600µg, 605µg, 610µg oder ähnliches beträgt. Für den Fachmann ist ersichtlich, dass vorstehend genannte Dosierungsvorschläge pro Einmalgabe nicht als auf die explizit angegebenen Zahlenwerte beschränkt anzusehen ist. Schwankungen um etwa ± 2,5µg, insbesondere Schwankungen im Dezimalbereich sind, wie für den Fachmann ersichtlich, ebenfalls umfasst. Bei diesen Dosierungsbereichen können die Wirkstoffe **1'** und **2** in den vorhergehend beschriebenen Gewichtsverhältnissen enthalten sein.

Beispielsweise und ohne den Umfang der Erfindung darauf zu beschränken, können die erfindungsgemäßen Kombinationen aus **1** und **2** eine solche Menge an Kation **1'** und Steroid **2** enthalten, dass pro Einmalgabe 16,5µg **1'** und 25µg **2**, 16,5µg **1**' und 25µg **2,** 16,5µg **1'** und 50µg **2,** 16,5µg **1'** und 100µg **2,** 16,5µg **1'** und 150µg **2,** 16,5µg **1'** und 200µg **2,** 16,5µg **1'** und 250µg **2**, 33,0µg **1**' und 25µg **2**, 33,0µg **1'** und 50µg **2,** 33,0µg **1'** und 100µg **2,** 33,0µg **1'** und 150µg **2**, 33,0µg **1'** und 200µg **2,** 33,0µg **1'** und 250µg **2,** 49,5µg **1'** und 25µg **2,** 49,5µg **1'** und 50µg **2,** 49,5µg **1'** und 100µg **2**, 49,5µg **1'** und 150µg **2,** 49,5µg **1'** und 200µg **2**, 49,5µg **1'** und 250µg **2**, 82,6µg **1'** und 25µg **2,** 82,6µg **1**' und 50µg **2**, 82,6µg **1'** und 100µg **2**, 82,6µg **1'** und 150µg **2**, 82,6µg **1'** und 200µg **2,** 82,6µg **1'** und 250µg **2**, 165,1µg **1**' und 25µg **2**, 165,1µg **1'** und 50µg **2,** 165,1µg **1**' und 50µg **2**, 165,1µg **1'** und 100µg **2**, 165,1µg **1'** und 150µg **2,** 165,1µg **1'** und 200µg **2**, 185,1µg **1'** und 250µg **2**, 206,4µg **1**' und 25µg **2**, 206.4µg **1'** und 50µg **2**, 206,4µg **1'** und 100µg **2**, 206,4µg **1'** und 150µg **2**, 206,4µg **1'** und 200µg **2,** 206,4µg **1'** und 250µg **2,** 412,8µg **1**' und 25µg **2,** 412,8µg **1'** und 50µg **2,** 412,8µg **1'** und 100µg **2,** 412,8µg **1'** und 150µg **2,** 412,8µg **1'** und 200µg **2,** 412,8µg **1'** und 250µg **2** enthalten sind.

Wird als eine erfindungsgemäß bevorzugte Kombination aus **1** und **2** die Wirkstoffkombination verwendet, in der als Salz **1** das Bromid verwendet wird und in der **2** für eines der vorstehend als bevorzugt offenbarten Steroide steht, entsprechen die vorstehen beispielhaft genannten pro Einmalgabe applizierten Wirkstoffmengen von **1'** und **2** den nachfolgenden pro Einmalgabe applizierten Wirkstoffmengen von **1'** und **2** den nachfolgenden pro Einmalgabe applizierten Mengen an **1** und **2:** 20µg **1** und 25µg **2,** 20µg **1** und 50µg **2,** 20µg **1** und 100µg **2,** 20µg **1** und 150µg **2,** 20µg **1** und 200µg **2,** 20µg **1** und 250µg **2,** 40µg **1** und 25µg **2,** 40µg **1** und 25µg **2,** 40µg **1** und 50µg **2,** 40µg **1** und 100µg **2,** 40µg **1** und 150µg **2,** 40µg **1** und 200µg **2,** 40µg **1** und 250µg **2**, 60µg **1** und 25µg **2,** 60µg **1** und 50µg **2,** 60µg **1** und 100µg **2,** 60µg **1** und 150µg **2,** 60µg **1** und 200µg **2,** 60µg **1** und 250µg **2,** 100µg **1** und 25µg **2,** 100µg **1** und 50µg **2,** 100µg **1** und 100µg **2,** 100µg **1** und 150µg **2**, 100µg **1** und 200µg **2,** 100µg **1** und 250µg **2**, 200µg **1** und 25µg **2**, 200µg **1** und 50µg **2**, 200µg **1** und 100µg **2,** 200µg **1** und 150µg **2,** 200µg **1** und 200µg **2,** 200µg **1** und 250µg **2**, 250µg **1** und 25µg **2**, 250µg **1** und 50µg **2,** 250µg **1** und 100µg **2,** 250µg **1** und 150µg **2**, 250µg **1** und 200µg **2,** 250µg **1** und 250µg **2,** 500µg **1** und 25µg **2,** 500µg **1** und 50µg **2,** 500µg **1** und 100µg **2,** 500µg **1** und 150µg **2**, 500µg **1** und 200µg **2**, 500µg **1** und 250µg **2**, enthalten sind.

Gleichzeitig kann das Verhältnis von **1** zu **3** 1:300 bis 30:1, bevorzugt von 1:230 bis 20:1, besonders bevorzugt von 1:150 bis 10:1, ferner bevorzugt von 1:50 bis 5:1, besonders bevorzugt von 1:35 bis 2:1 liegen.

Die erfindungsgemäßen Wirkstoffkombinationen können **1'** und **3'** im Falle von beispielsweise Formoterol in Gewichtsverhältnissen enthalten, die beispielsweise in einem Bereich von etwa 1:10 bis 300:1, bevorzugt 1:5 bis 200:1, bevorzugt 1:3 bis 150:1, besonders bevorzugt von 1:2 bis 100:1 liegen.

Beispielsweise und ohne den Umfang der Erfindung darauf zu beschränken, können bevorzugte erfindungsgemäße Kombinationen aus **1** und **3** das pharmakologisch aktive Kation **1**' und Formoterol **3'** in den folgenden Gewichtsverhältnissen enthalten: 1:5, 1:4, 1:3, 1:2, 1:1, 2:1, 3:1, 4:1, 5:1, 6:1, 7:1, 8:1, 9:1, 10:1, 11:1, 12:1, 13:1, 14:1, 15:1, 16:1, 17:1, 18:1, 19:1, 20:1, 21:1, 22:1, 23:1, 24:1, 25:1, 26:1, 27:1, 28:1, 29:1, 30:1, 31:1, 32:1, 33:1, 34:1, 35:1, 36:1, 37:1, 38:1, 39:1, 40:1, 41:1, 42:1, 43:1, 44:1, 45:1, 46:1, 47:1, 48:1, 49:1, 50:1, 51:1, 52:1, 53:1, 54:1, 55:1 , 56:1, 57:1, 58:1, 59:1, 60:1, 61:1, 62:1, 63:1, 64:1, 65:1, 66:1, 67:1, 68:1, 69:1, 70:1, 71:1, 72:1, 73:1, 74:1, 75:1, 76:1, 77:1, 78:1, 79:1, 80:1, 81:1, 82:1, 83:1, 84:1, 85:1, 86:1, 87:1, 88:1, 89:1, 90:1, 91:1, 92:1, 93:1, 94:1, 95:1, 96:1, 97:1, 98:1, 99:1, 100:1.

Die Anwendung der erfindungsgemäßen Arzneimittel enthaltend die Kombinationen aus **1** und **3** erfolgt üblicherweise so, daß das pharmakologisch aktive Kation **1'** und Formoterol **3'** gemeinsam in Dosierungen von 5 bis 5000µg, bevorzugt von 10 bis 2000µg, besonders bevorzugt von 15 bis 1000µg, ferner bevorzugt von 20 bis 800µg, erfindungsgemäß bevorzugt von 30 bis 600µg, bevorzugt von 40 bis 500µg enthalten sind.

Beispielsweise enthalten erfindungsgemäße Kombinationen aus **1** und **3** eine solche Menge an Kation **1**' und Formoterol **3'**, daß die Gesamtdosierung pro Einmalgabe etwa 10µg, 15µg, 20µg, 25µg, 30µg, 35µg, 45µg, 50µg, 55µg, 60µg, 65µg, 70µg, 75µg, 80µg, 85µg, 90µg, 95µg, 100µg, 105µg, 110µg, 115µg, 120µg, 125µg, 130µg, 135µg, 140µg, 145µg, 150µg, 155µg, 160µg, 165µg, 170µg, 175µg, 180µg, 185µg, 190µg, 195µg, 200µg, 205µg, 210µg, 215µg, 220µg, 225µg, 230µg, 235µg, 240µg, 245µg, 250µg, 255µg, 260µg, 265µg, 270µg, 275µg 280µg, 285µg, 290µg, 295µg, 300µg, 305µg, 310µg, 315µg, 320µg, 325µg, 330µg, 335µg, 340µg, 345µg, 350µg, 355µg, 360µg, 365µg, 370µg, 375µg, 380µg, 385µg, 390µg, 395µg, 400µg, 405µg, 410µg, 415µg, 420µg, 425µg, 430µg, 435µg, 440µg, 445µg, 450µg, 455µg, 460µg, 465µg, 470µg, 475µg, 480µg, 485µg, 490µg, 495µg, 500µg, 505µg, 510µg, 515µg, 520µg, 525µg, 530µg, 535µg, 540µg, 545µg, 550µg, 555µg, 560µg, 565µg, 570µg, 575µg, 580µg, 585µg, 590µg, 595µg, 600µg, oder ähnliches beträgt. Für den Fachmann ist ersichtlich, daß vorstehend genannte Dosierungsvorschläge pro Einmalgabe nicht als auf die explizit angegebenen Zahlenwerte beschränkt anzusehen ist. Schwankungen um etwa ± 2,5 µg, insbesondere Schwankungen im Dezimalbereich sind, wie für den Fachmann ersichtlich, ebenfalls umfaßt. Bei diesen Dosierungsbereichen sind die Wirkstoffe **1'** und **3'** in den vorhergehend beschriebenen Gewichtsverhältnissen enthalten.

Beispielsweise und ohne den Umfang der Erfindung darauf zu beschränken, können die erfindungsgemäßen Kombinationen aus **1** und **3** eine solche Menge an Kation **1'** und Formoterol **3'** enthalten, daß pro Einmalgabe beispielsweise 8,3µg **1'** und 2,5µg **3'**, 8,3µg **1'** und 4,9µg **3**', 8,3µg **1'** und 9,8µg **3'**, 8,3µg **1**' und 14,7µg **3'**, 8,3µg **1'** und 19,6µg **3'**, 8,3µg **1'** und 24,4µg **3'**, 16,5µg **1'** und 2,5µg **3**', 16,5µg **1**' und 4,9µg **3'**, 16,5µg **1'** und 9,8µg **3'**, 16,5µg **1'** und 14,7µg **3'**, 16,5µg **1'** und 19,6µg **3'**, 16,5µg **1'** und 24,4µg **3'**, 33,µg **1'** und 2,5µg **3'**, 33,0µg **1'** und 4,9µg **3'**, 33,0µg **1'** und 9,8µg **3'**, 33,0µg **1'** und 14,7µg **3'**, 33,0µg **1'** und 19,6µg **3'**, 33,0µg **1'** und 24,4µg **3'**, 49,5µg **1'** und 2,5µg **3'**, 49,5µg **1**' und 4,9µg **3'**, 49,5µg **1'** und 9,8µg **3'**, 49,5µg **1'** und 14,7µg **3'**, 49,5µg **1**' und 19,6µg **3'**, 49,5µg **1'** und 24,4µg **3'**, 82.6µg **1**' und 2,5µg **3'**, 82,6µg **1'** und 4,9µg **3'**, 82,6µg **1'** und 9,8µg **3'**, 82,6µg **1'** und 14,7µg **3'**, 82,6µg **1'** und 19,6µg **3',** 82,6µg **1'** und 24,4µg **3'**, 165,1µg **1'** und 2,5µg **3'**, 165,1µg **1'** und 4,9µg **3'**, 165,1µg **1'** und 9,8µg **3'**, 165,1µg **1'** und 14,7µg **3'**, 165,1µg **1'** und 19,6µg **3'**, 165,1µg **1'** und 24,4µg **3',** 206,4µg **1'** und 2,5µg **3',** 206,4µg **1*'*** und 4,9µg **3',** 206,4µg **1'** und 9,8µg **3'**, 206,4µg **1'** und 14,7µg **3',** 206,4µg **1'** und 19,6µg **3',** 206,4µg **1'** und 24,4µg **3',** 412,8µg **1'** und 2,5µg **3',** 412,8µg **1'** und 4,9µg **3',** 412,8µg **1'** und 9,8µg **3',** 412,8µg **1'** und 14,7µg **3',** 412,8µg **1'** und 19,6µg **3',** 412,8µg **1'** und 24,4µg **3'** enthalten sind.

Wird als eine erfindungsgemäß bevorzugte Kombination aus **1** und **3** die Wirkstoffkombination verwendet, in der das Salz **1** das Bromid darstellt und in der **3** für Formoterolfumarat steht, entsprechen die vorstehend beispielhaft genannten pro Einmalgabe applizierten Wirkstoffmengen von **1'** und **3'** den nachfolgenden pro Einmalgabe applizierten Mengen an **1** und **3**: 10µg **1** und 2,9µg **3**, 10µg **1** und 5,7µg **3,** 10µg **1** und 11,5µg **3**, 10µg **1** und 17,2µg **3**, 10µg **1** und 22,9µg **3**, 10µg **1** und 28,5µg **3**, 20µg **1** und 2,9µg **3**, 20µg **1** und 5,7µg **3**, 20µg **1** und 11,5µg **3**, 20µg **1** und 17,2µg **3**, 20µg **1** und 22,9µg **3**, 20µg **1** und 28,5µg **3**, 40µg **1** und 2,9µg **3**, 40µg **1** und 5,7µg **3**, 40µg **1** und 11,5µg **3**, 40µg **1** und 17,2µg **3**, 40µg **1** und 22,9µg **3**, 40µg **1** und 28,5µg **3**, 60µg **1** und 2,9µg **3**, 60µg **1** und 5,7µg **3**, 60µg **1** und 11,5µg **3**, 60µg **1** und 17,2µg **3**, 60µg **1** und 22,9µg **3,** 60µg **1** und 28,5µg **3**, 100µg **1** und 2,9µg **3**, 100µg **1** und 5,7µg **3**, 100µg **1** und 11,5µg **3**, 100µg **1** und 17,2µg **3**, 100µg **1** und 22,9µg **3,** 100µg **1** und 28,5µg **3,** 200µg **1** und 2,9µg **3,** 200µg **1** und 5,7µg **3,** 200µg **1** und 11,5µg **3,** 200µg **1** und 17,2µg **3,** 200µg **1** und 22,9µg **3,** 200µg **1** und 28,5µg **3,** 250µg **1** und 2,9µg **3**, 250µg **1** und 5,7µg **3**, 250µg **1** und 11,5µg **3**, 250µg **1** und 17,2µg **3,** 250µg **1** und 22,9µg **3**, 250µg **1** und 28,5µg **3,** 500µg **1** und 2,9µg **3**, 500µg **1** und 5,7µg **3,** 500µg **1** und 11,5µg **3,** 500µg **1** und 17,2µg **3,** 500µg **1** und 22,9µg **3,** 500µg **1** und 28,5mg **3.**

Wird als eine erfindungsgemäß bevorzugte Kombination aus **1** und **3**, die Wirkstoffkombination verwendet, in der **3** für Formoterolfumaratdihydrat steht und das Salz **1** das Bromid darstellt, entsprechen die vorstehend beispielhaft genannten pro Einmalgabe applizierten Wirkstoffmengen von **1'** und **3'** den nachfolgenden pro Einmalgabe applizierten Mengen an **1** und **3:** 10µg **1** und 3µg **3,** 10µg **1** und 6µg **3,** 10µg **1** und 12µg **3,** 10µg **1** und 18µg **3**, 10µg **1** und 24µg **3**, 10µg **1** und 30µg **3**, 20µg **1** und 3µg **3**, 20µg **1** und 6µg **3**, 20µg **1** und 12µg **3**, 20µg **1** und 18µg **3**, 20µg **1** und 24µg **3**, 20µg **1** und 30µg **3**, 40µg **1** und 3µg **3,** 40µg **1** und 6µg **3,** 40µg **1** und 12µg **3,** 40µg **1** und 18µg **3,** 40µg **1** und 24µg **3,** 40µg **1** und 30µg **3**, 60µg **1** und 3µg **3**, 60µg **1** und 6µg **3**, 60µg **1** und 12µg **3**, 60µg **1** und 18µg **3**, 60µg **1** und 24µg **3**, 60µg **1** und 30µg **3,** 100µg **1** und 3µg **3**, 100µg **1** und 6µg **3**, 100µg **1** und 12µg **3**, 100µg **1** und 18µg **3**, 100µg **1** und 24µg **3**, 100µg **1** und 30µg **3**, 200µg **1** und 3µg **3**, 200µg **1** und 6µg **3**, 200µg **1** und 12µg **3**, 200µg **1** und 18µg **3**, 200µg **1** und 24µg **3**, 200µg **1** und 30µg **3**, 250µg **1** und 3µg **3**, 250µg **1** und 6µg **3**, 250µg **1** und 12µg **3**, 250µg **1** und 18µg **3**, 250µg **1** und 24µg **3**, 250µg **1** und 30µg **3**, 500µg **1** und 3µg **3**, 500µg **1** und 6µg **3**, 500µg **1** und 12µg **3**, 500µg **1** und 18µg **3**, 500µg **1** und 24µg **3**, 500µg **1** und 30µg **3**.

Die erfindungsgemäßen Wirkstoffkombinationen können **1'** und **3'** im Falle von beispielsweise Salmeterol in Gewichtsverhältnissen enthalten, die beispielsweise in einem Bereich von etwa 1:30 bis 400:1, bevorzugt 1:25 bis 200:1, bevorzugt 1:20 bis 100:1, besonders bevorzugt von 1:15 bis 50:1 liegen.

Beispielsweise und ohne den Umfang der Erfindung darauf zu beschränken, können bevorzugte erfindungsgemäße Kombinationen aus **1** und **3** das Kation **1'** und Salmeterol **3'** in den folgenden Gewichtsverhältnissen enthalten: 1:15, 1:14, 1:13, 1:12, 1:11, 1:10, 1:9, 1:8, 1:7, 1:6, 1:5, 1:4, 1:3, 1:2, 1:1, 2:1, 3:1, 4:1, 5:1, 6:1, 7:1, 8:1, 9:1, 10:1, 11:1, 12:1, 13:1, 14:1, 15:1, 16:1, 17:1, 18:1, 19:1, 20:1, 21:1, 22:1, 23:1, 24:1, 25:1, 26:1, 27:1, 28:1, 29:1, 30:1, 31:1, 32:1, 33:1, 34:1. 35:1.

Die Anwendung der erfindungsgemäßen Arzneimittel enthaltend die Kombinationen aus **1** und **3** erfolgt üblicherweise so, daß das Kation **1'** und Salmeterol **3'** gemeinsam in Dosierungen von 5 bis 5000µg, bevorzugt von 10 bis 2000µg, besonders bevorzugt von 15 bis 1000µg, ferner bevorzugt von 20 bis 800µg, erfindungsgemäß bevorzugt von 30 bis 750µg, bevorzugt von 40 bis 700µg enthalten sind.

Beispielsweise enthalten erfindungsgemäße Kombinationen aus **1** und **3** eine solche Menge an **1'** und Salmeterol **3'**, daß die Gesamtdosierung pro Einmalgabe etwa 15µg, 20µg, 25µg, 30µg, 35µg, 45µg, 50µg, 55µg, 60µg, 65µg, 70µg, 75µg, 80µg, 85µg, 90µg, 95µg, 100µg, 105µg, 110µg, 115µg, 120µg, 125µg, 130µg, 135µg, 140µg, 145µg, 150µg, 155µg, 160µg, 165µg, 170µg, 175µg, 180µg, 185µg, 190µg, 195µg, 200µg, 205µg, 210µg, 215µg, 220µg, 225µg, 230µg, 235µg, 240µg, 245µg, 250µg, 255µg, 260µg, 265µg, 270µg, 275µg, 280µg, 285µg, 290µg, 295µg, 300µg, 305µg, 310µg, 315µg, 320µg, 325µg, 330µg, 335µg, 340µg, 345µg, 350µg, 355µg, 360µg, 365µg, 370µg, 375µg, 380µg, 385µg, 390µg, 395µg, 400µg, 405µg, 410µg. 415µg, 420µg, 425µg, 430µg, 435µg, 440µg, 445µg, 450µg, 455µg, 460µg, 465µg, 470µg, 475µg, 480µg, 485µg, 490µg, 495µg, 500µg, 505µg, 510µg, 515µg, 520µg, 525µg, 530µg, 535µg, 540µg, 545µg, 550µg, 555µg, 560µg, 565µg, 570µg, 575µg, 580µg, 585µg, 59µg, 595µg, 600µg, 605µg, 610µg, 615µg, 620µg, 625µg, 630µg, 635µg, 640µg, 645µg, 650µg, 655µg, 660µg, 665µg, 670µg, 675µg, 680µg, 685µg, 690µg, 695µg, 700µg oder ähnliches beträgt. Für den Fachmann ist ersichtlich, daß vorstehend genannte Dosierungsvorschläge pro Einmalgabe nicht als auf die explizit angegebenen Zahlenwerte beschränkt anzusehen ist. Schwankungen um etwa ± 2,5 µg, insbesondere Schwankungen im Dezimalbereich sind, wie für den Fachmann ersichtlich, ebenfalls umfaßt. Bei diesen Dosierungsbereichen sind die Wirkstoffe **1'** und **3'** in den vorhergehend beschriebenen Gewichtsverhältnissen enthalten.

Beispielsweise und ohne den Umfang der Erfindung darauf zu beschränken, können die erfindungsgemäßen Kombinationen aus **1** und **3** eine solche Menge an Kation **1**' und Salmeterol **3'** enthalten, daß pro Einmalgabe beispielsweise 8,3µg **1'** und 12,5µg **3',** 8,3µg **1'** und 25µg **3',** 8,3µg **1'** und 50µg **3',** 8,3µg **1'** und 75µg **3',** 8,3µg **1'** und 100µg **3',** 8,3µg **1'** und 200µg **3',** 16,5µg **1'** und 12,5µg **3'**, 16,5µg **1'** und 25µg **3'**, 16,5µg **1'** und 50µg **3',** 16,5µg **1'** und 75µg **3',** 16,5µg **1'** und 100µg **3',** 16,5µg **1'** und 200µg **3',** 33,0µg **1'** und 12,5µg **3',** 33,0µg **1'** und 25µg **3',** 33,0µg **1'** und 50µg **3',** 33,0µg **1**' und 75µg **3**', 33,0µg **1'** und 100µg **3',** 33,0µg **1**' und 200µg **3',** 49,5µg **1**' und 12,5µg **3**', 49,5µg **1**' und 25µg **3**', 49,5µg **1**' und 50µg **3**', 49,5µg **1'** und 75µg **3**', 49,5µg **1**' und 100µg **3',** 49,5µg **1'** und 200µg **3'**, 82,6µg **1**' und 12,5µg **3**', 82,6µg **1**' und 25µg **3',** 82,6µg **1**' und 50µg **3',** 82,6µg **1'** und 75µg **3',** 82,6µg **1'** und 100µg **3'**, 82,6µg **1'** und 200µg **3'**, 165,1µg **1'** und 12,5µg **3'**, 165,1µg **1'** und 25µg **3'**, 165,1µg **1'** und 50µg **3'**, 165,1µg **1'** und 75µg **3'**, 165,1µg **1'** und 100µg **3'**, 165,1µg **1'** und 200µg **3'**, 206,4µg **1'** und 12,5µg **3'**, 206,4µg **1'** und 25µg **3'**, 206,4µg **1'** und 50µg **3'**, 206,4µg **1'** und 75µg **3'**, 206,4µg **1'** und 100µg **3'**, 206,4µg **1'** und 200µg **3'**, 412,8µg **1'** und 12,5µg **3'**, 412,8µg **1'** und 25µg **3'**, 412,8µg **1'** und 50µg **3'**, 412,8µg **1'** und 75µg **3'**, 412,8µg **1'** und 100µg **3'**, 412,8µg **1'** und 200µg **3'** enthalten sind.

Wird als eine erfindungsgemäß bevorzugte Kombination aus **1** und **3** die Wirkstoffkombination verwendet, in der als Salz **1** das Bromid verwendet wird und in der **3** für Salmeterolxinafoat steht, entsprechen die vorstehend beispielhaft genannten pro Einmalgabe applizierten Wirkstoffmengen von **1'** und **3'** den nachfolgenden pro Einmalgabe applizierten Mengen an **1** und **3**: 10µg **1** und 18,2µg **3**, 10µg **1** und 36,3µg **3**, 10µg **1** und 72,6µg **3,** 10µg **1** und 108,9µg **3**, 10µg **1** und 145,2µg **3**, 10µg **1** und 290,4µg **3**, 20µg **1** und 18,2µg **3**, 20µg **1** und 36,3µg **3**, 20µg **1** und 72,6µg **3**, 20µg **1** und 108,9µg **3**, 20µg **1** und 145,2µg **3**, 20µg **1** und 290,4µg **3,** 40µg **1** und 18,2µg **3**, 40µg **1** und 38,3µg **3**, 40µg **1** und 72,6µg **3**, 40µg **1** und 108,9µg **3,** 40µg **1** und 145,2µg **3**, 40µg **1** und 290,4µg **3**, 60µg **1** und 18,2µg **3**, 60µg **1** und 36,3µg **3**, 60µg **1** und 72,6µg **3**, 60µg **1** und 108,9µg **3**, 60µg **1** und 145,2µg **3**, 60µg **1** und 290,4µg **3,** 100µg **1** und 18,2µg **3**, 100µg **1** und 36,3µg **3**, 100µg **1** und 72,6µg **3,** 100µg **1** und 108,9µg **3**, 100µg **1** und 145,2µg **3**, 100µg **1** und 290,4µg **3,** 200µg **1** und 18,2µg **3**, 200µg **1** und 36,3µg **3**, 200µg **1** und 72,6µg **3**, 200µg **1** und 108,9µg **3,** 200µg **1** und 145,2µg **3**, 200µg **1** und 290,4µg **3,** 250µg **1** und 18,2µg **3**, 280µg **1** und 36,3µg **3**, 250µg **1** und 72,6µg **3**, 250µg **1** und 108,9µg **3,** 250µg **1** und 145,2µg **3**, 250µg **1** und 290,4µg **3**, 500µg **1** und 18,2µg **3**, 500µg **1** und 36,3µg **3**, 500µg **1** und 72,6µg **3**, 500µg **1** und 108,9µg **3,** 500µg **1** und 145,2µg **3**, 500µg **1** und 290,4µg **3.**

In analoger Art und Weise sind die pro Einmalgabe applizierten Wirkstoffmengen der erfindungsgemäßen Arzneimittelkombinationen berechenbar, wenn statt des Salmeterolxinafoats die als ebenfalls erfindungsgemäß bevorzugt zum Einsatz gelangenden Verbindungen **3** Salmeterol-4-Phenylzimtsäuresalz (4-Phenylcinnamat) und Salmeterol-5-(2,4-difluorphenyl)salicylsäuresalz (5-(2,4-difluorphenyl)salicylat; Diflunisal) verwendet werden.

Die Applikation der erfindungsgemäßen Wirkstoffkombinationen aus **1**, **2** und **3** erfolgt bevorzugt auf inhalativem Wege. Hierzu müssen die Bestandteile **1**, **2** und **3** in inhalierbaren Darreichungsformen bereitgestellt werden. Als inhalierbare Darreichungsformen kommen erfindungsgemäß Inhalationspulver, treibgashaltige Dosieraerosole oder treibgasfreie Inhalationslösungen in Betracht. Erfindungsgemäße Inhalationspulver enthaltend die Wirkstoffkombination aus **1, 2** und **3** können allein aus den genannten Wirkstoffen oder aus einem Gemisch der genannten Wirkstoffe mit physiologisch verträglichen Hilfsstoffen bestehen. Gegebenenfalls wird im Rahmen der vorliegenden Erfindung statt des Begriffs Hilfsstoffs auch der Begriff Trägerstoff verwendet. Im Rahmen der vorliegenden Erfindung sind von dem Begriff treibgasfreie Inhalationslösungen auch Konzentrate oder sterile, gebrauchsfertige Inhalationslösungen umfaßt. Die erfindungsgemäßen Darreichungsformen können die Wirkstoffkombination aus **1**, **2** und **3** entweder gemeinsam in einer, in zwei oder in drei getrennten Darreichungsformen enthalten. Diese im Rahmen der vorliegenden Erfindung einsetzbaren Darreichungsformen werden im nachfolgenden Teil der Beschreibung detailliert beschrieben.

### A) Inhalationspulver enthaltend die erfindungsgemäßen Wirkstoffkombinationen aus 1, 2 und 3:

Die erfindungsgemäßen Inhaltionspulver können **1**, **2** und **3** entweder allein oder im Gemisch mit geeigneten physiologisch unbedenklichen Hilfsstoffen enthalten.

Sind die Wirkstoffe **1**, **2** und **3** im Gemisch mit physiologisch unbedenklichen Hilfsstoffen enthalten, können zur Darstellung dieser erfindungsgemäßen inhalationspulver die folgenden physiologisch unbedenklichen Hilfsstoffe zur Anwendung gelangen: Monosaccharide (z.B. Glucose oder Arabinose), Disaccharide (z.B. Lactose, Saccharose, Maltose), Oligo- und Polysaccharide (z.B. Dextrane), Polyalkohole (z.B. Sorbit, Mannit, Xylit), Salze (z.B. Natriumchlorid, Calciumcarbonat) oder Mischungen dieser Hilfsstoffe. Bevorzugt gelangen Mono- oder Disaccharide zur Anwendung, wobei die Verwendung von Lactose oder Glucose, insbesondere, aber nicht ausschließlich in Form ihrer Hydrate, bevorzugt ist. Als besonders bevorzugt im Sinne der Erfindung gelangt Lactose, höchst bevorzugt Lactosemonohydrat als Hilfsstoff zur Anwendung.

Die Hilfsstoffe weisen im Rahmen der erfindungsgemäßen Inhaltionspulver eine maximale mittlere Teilchengröße von bis zu 250µm, bevorzugt zwischen 10 und 150µm, besonders bevorzugt zwischen 15 und 80µm auf. Gegebenenfalls kann es sinnvoll erscheinen, den vorstehend genannten Hilfststoffen feinere Hilfsstofffraktionen mit einer mittleren Teilchengröße von 1 bis 9µm beizumischen. Letztgenannte feinere Hilfsstoffe sind ebenfalls ausgewählt aus der vorstehend genannten Gruppe an einsetzbaren Hilfsstoffen. Schließlich wird zur Herstellung der erfindungsgemäßen Inhaltionspulver mikronisierter Wirkstoff **1**, **2** und **3**, vorzugsweise mit einer mittleren Teilchengröße von 0,5 bis 10µm, besonders bevorzugt von 1 bis 5µm, der Hilfsstoffmischung beigemischt. Verfahren zur Herstellung der erfindungsgemäßen Inhaltionspulver durch Mahlen und Mikronisieren sowie durch abschließendes Mischen der Bestandteile sind aus dem Stand der Technik bekannt. Die erfindungsgemäßen lnhaltionspulver können entweder in Form einer einzigen Pulvermischung, die sowohl **1** als auch **2** und **3** enthält oder in Form von separaten Inhalationspulvern, die lediglich **1**, **2** beziehungsweise **3** enthalten bereitgestellt und appliziert werden.

Die erfindungsgemäßen Inhalationspulver können mittels aus dem Stand der Technik bekannten Inhalatoren appliziert werden.
Erfindungsgemäße Inhalationspulver, die neben **1**, **2** und **3** ferner einen oder mehrere physiologisch unbedenkliche Hilfsstoffe enthalten, können beispielsweise mittels Inhalatoren appliziert werden, die eine einzelne Dosis aus einem Vorrat mittels einer Meßkammer, wie er in der US 4570630A beschrieben wird, oder über andere apparative Vorrichtungen, wie sie in der DE 36 25 685 A beschrieben werden, dosieren. Vorzugsweise werden die erfindungsgemäßen Inhalationspulver, die neben **1**, **2** und **3** physiologisch unbedenkliche Hilfsstoffe enthalten, allerdings in Kapseln abgefüllt (zu sogenannten Inhaletten), die in Inhalatoren wie beispielsweise in der WO 94/28958 beschrieben, zur Anwendung gelangen.

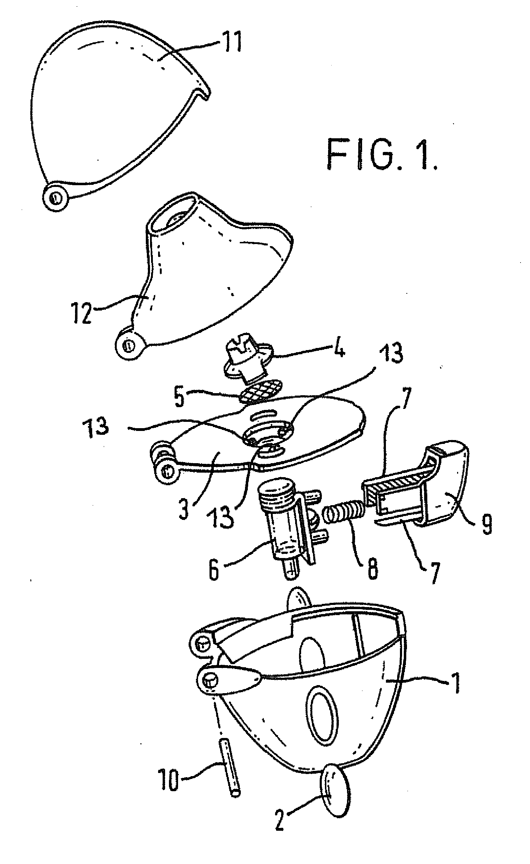

Ein zur Anwendung der erfindungsgemäßen Arzneimittelkombination in Inhaletten besonders bevorzugter Inhalator ist Figur 1 zu entnehmen.
Dieser Inhalator (Handihaler) für die Inhalation pulverförmiger Arzneimittel aus Kapseln ist gekennzeichnet durch ein Gehäuse 1, enthaltend zwei Fenster 2, ein Deck 3, in dem sich Lufteinlaßöffnungen befinden und welches mit einem über ein Siebgehäuse 4 befestigten Sieb 5 versehen ist, eine mit Deck 3 verbundene Inhalationskammer 6, an der ein mit zwei geschliffenen Nadeln 7 versehener, gegen eine Feder 8 beweglicher Drücker 9 vorgesehen ist, sowie ein über eine Achse 10 klappbar mit dem Gehäuse 1, dem Deck 3 und einer Kappe 11 verbundenes Mundstück 12 sowie Luftdurchlaßlöcher 13 zur Einstellung des Ströumungswiderstands.

Sollen die erfindungsgemäßen Inhalationspulver im Sinne der vorstehend gennannten bevorzugten Anwendung in Kapseln (Inhaletten) abgefüllt werden, bieten sich Füllmengen von 1 bis 30mg, bevorzugt von 3 bis 20mg, bevorzugt 5 bis 10 mg Inhalationspulver pro Kapsel an. Diese enthalten erfindungsgemäß entweder gemeinsam oder jeweils die bereits vorstehend für **1, 2** und **3** genannten Dosierungen pro Einmalgabe.

### B) Treibgashaltige Inhalationsaerosole enthaltend die erfindungsgemäßen Wirkstoffkombinationen aus 1, 2 und 3 :

Erfindungsgemäße treibgashaltige Inhaltionsaerosole können **1, 2** und **3** im Treibgas gelöst oder in dispergierter Form enthalten. Hierbei können **1, 2** und **3** in getrennten Darreichungsformen oder in einer gemeinsamen Darreichungsform enthalten sein, wobei **1**, **2** und **3** entweder jeweils gelöst, jeweils dispergiert oder gegebenenfalls nur eine oder zwei der Komponenten gelöst und die andere beziehungsweise die anderen Komponenten dispergiert enthalten sein können.
Die zur Herstellung der erfindungsgemäßen Inhaltionsaerosole einsetzbaren Treibgase sind aus dem Stand der Technik bekannt. Geeignete Treibgase sind ausgewählt aus der Gruppe bestehend aus Kohlenwasserstoffen wie n-Propan, n-Butan oder Isobutan und Halogenkohlenwasserstoffen wie fluorierten Derivaten des Methans, Ethans, Propans, Butans, Cyclopropans oder Cyclobutans. Die vorstehend genannten Treibgase können dabei allein oder in Mischungen derselben zur Verwendnung kommen. Besonders bevorzugte Treibgase sind halogenierte Alkanderivate ausgewählt aus TG134a und TG227 und Mischungen derselben.

Die erfindungsgemäßem treibgashaltigen Inhalationsaerosole können ferner weitere Bestandteile wie Kosolventien, Stabilisatoren, oberflächenaktive Mittel (surfactants), Antioxidantien, Schmiermittel, Konservierungsmittel sowie Mittel zur Einstellung des pH-Werts enthalten. All diese Bestandteile sind im Stand der Technik bekannt.

Die erfindungsgemäßen treibgashaltigen Inhaltionsaerosole können bis zu 5 Gew-% an Wirkstoff **1**, **2** und/oder **3** enthalten. Erfindungsgemäße Aerosole enthalten beispielsweise 0,002 bis 5 Gew-%, 0,01 bis 3 Gew-%, 0,015 bis 2 Gew-%, 0,1 bis 2 Gew-%, 0,5 bis 2 Gew-% oder 0,5 bis 1 Gew-% an Wirkstoff **1**, **2** und/oder **3**.

Liegen die Wirkstoffe **1, 2** und/oder **3** in dispergierter Form vor, weisen die Wirkstoffteilchen bevorzugt eine mittlere Teilchengröße von bis zu 10 µm, bevorzugt von 0,1 bis 5 µm, besonders bevorzugt von 1 bis 5 µm auf.

Die vorstehend genannten erfindungsgemäßen treibgashaltigen Inhaltionaerosole können mittels im Stand der Technik bekannten Inhalatoren (MDIs = metered dose inhalers) appliziert werden. Dementsprechend betrifft ein weiterer Aspekt der vorliegenden Erfindung Arzneimittel in Form von wie vorstehend beschriebenen treibgashaltigen Aerosolen in Verbindung mit einem oder mehreren zur Verabreichung dieser Aerosole geeigneten Inhalatoren. Ferner betrifft die vorliegende Erfindung Inhalatoren, dadurch gekennzeichnet, daß sie vorstehend beschriebene erfindungsgemäße treibgashaltige Aerosole enthalten.
Die vorliegende Erfindung betrifft ferner Kartuschen, die ausgestattet mit einem geeigneten Ventil in einem geeigneten Inhalator zur Anwendung gelangen können und die eine der vorstehend genannten erfindungsgemäßen treibgashaltigen lnhalationsaerosole enthalten. Geeignete Kartuschen und Verfahren zur Abfüllung dieser Kartuschen mit den erfindungsgemäßen treibgashaltigen Inhaltionsaerosolen sind aus dem Stand der Technik bekannt.
C) Treibgasfreie Inhaltionslösungen oder Suspensionen enthaltend die erfindungsgemäßen Wirkstoffkombinationen aus **1**, **2** und **3:**
   Besonders bevorzugt erfolgt die Applikation der erfindungsgemäßen Wirkstoffkombination in Form von treibgasfreien Inhalationslösungen und lnhaltionssuspensionen. Als Lösungsmittel kommen hierzu wässrige oder alkoholische, bevorzugt ethanolische Lösungen in Betracht. Das Lösungsmittel kann ausschließlich Wasser sein oder es ist ein Gemisch aus Wasser und Ethanol. Der relative Anteil an Ethanol gegenüber Wasser ist nicht begrenzt, bevorzugt liegt die maximale Grenze jedoch bei bis 70 Volumenprozent, insbesondere bei bis zu 60 Volumenprozent und besonders bevorzugt bei bis zu 30 Volumenprozent. Die restlichen Volumenprozente werden von Wasser aufgefüllt. Die **1**, **2** und **3** , getrennt oder gemeinsam enthaltenden Lösungen oder Suspensionen werden mit geeigneten Säuren auf einen pH-Wert von 2 bis 7, bevorzugt von 2 bis 5 eingestellt. Zur Einstellung dieses pH-Werts können Säuren ausgewählt aus anorganischen oder organischen Säuren Verendung finden. Beispiele für besonders geeignete anorganische Säuren sind Salzsäure, Bromwasserstoffsäure, Salpetersäure, Schwefelsäure und/oder Phosphorsäure. Beispiele für besonders geeignete organische Säuren sind: Ascorbinsäure, Zitronensäure, Äpfelsäure, Weinsäure, Maleinsäure, Bernsteinsäure, Fumarsäure, Essigsäure, Ameisensäure und/oder Propionsäure und andere. Bevorzugte anorganische Säuren sind Salzsäure, Schwefelsäure. Es können auch die Säuren verwendet werden, die bereits mit einem der Wirkstoffe ein Säureadditionssalz bilden. Unter den organischen Säuren sind Ascorbinsäure, Fumarsäure und Zitronensäure bevorzugt. Gegebenenfalls können auch Gemische der genannten Säuren eingesetzt werden, insbesondere in Fällen von Säuren, die neben ihren Säuerungseigenschaften auch andere Eigenschaften, z.B. als Geschmackstoffe, Antioxidantien oder Komplexbildner besitzen, wie beispielsweise Zitronensäure oder Ascorbinsäure. Erfindungsgemäß besonders bevorzugt wird Salzsäure zur Einstellung des pH-Werts verwendet.

Erfindungsgemäß kann in der vorliegenden Formulierung auf den Zusatz von Editinsäure (EDTA) oder einem der bekannten Salze davon, Natriumedetat, als Stabilisator oder Komplexbildner verzichtet werden.
Andere Ausführungsformen beinhalten diese Verbindung(en).
In einer solchen bevorzugten Ausführungsform liegt der Gehalt bezogen auf Natriumedetat unter 100 mg /100ml, bevorzugt unter 50 mg/100ml, besonders bevorzugt unter 20 mg/100ml. Generell sind solche Inhaltionslösungen bevorzugt, in denen der Gehalt an Natriumedetat bei 0 bis 10mg/100ml liegt.

Den erfindungsgemäßen treibgasfreien Inhaltionslösungen können Co-Solventien und/oder weitere Hilfsstoffe zugesetzt werden.
Bevorzugte Co-Solventien sind solche, die Hydroxylgruppen oder andere polare Gruppen enthalten, beispielsweise Alkohole - insbesondere Isopropylalkohol, Glykole - insbesondere Propylenglykol, Polyethylenglykol, Polypropylenglykol, Glykolether, Glycerol, Polyoxyethylenalkohole und Polyoxyethylen-Fettsäureester.
Unter Hilfs- und Zusatzstoffen wird in diesem Zusammenhang jeder physiologisch verträgliche Stoff verstanden, der kein Wirkstoff ist, aber zusammen mit dem (den) Wirkstoff(en) in dem physiologisch geeigneten Lösungsmittel formuliert werden kann, um die qualitativen Eigenschaften der Wirkstoffformulierung zu verbessern. Bevorzugt entfalten diese Stoffe keine oder im Kontext mit der angestrebten Therapie keine nennenswerte oder zumindest keine unerwünschte pharmakologische Wirkung. Zu den Hilfs- und Zusatzstoffen zählen z.B. oberflächenaktive Stoffe, wie z.B. Sojalecithin, Ölsäure, Sorbitanester, wie Polysorbate, Polyvinylpyrrolidon sonstige Stabilisatoren, Komplexbildner, Antioxidantien und/oder Konservierungsstoffe, die die Verwendungsdauer der fertigen Arzneimittelformulierung gewährleisten oder verlängern, Geschmackstoffe, Vitamine und/oder sonstige dem Stand der Technik bekannte Zusatzstoffe. Zu den Zusatzstoffen zählen auch physiologisch unbedenkliche Salze wie beispielsweise Natriumchlorid als Isotonantien.

Zu den bevorzugten Hilfsstoffen zählen Antioxidantien, wie beispielsweise Ascorbinsäure, sofern nicht bereits für die Einstellung des pH-Werts verwendet, Vitamin A, Vitamin E, Tocopherole und ähnliche im menschlichen Organismus vorkommende Vitamine oder Provitamine.
Konservierungsstoffe können eingesetzt werden, um die Formulierung vor Kontamination mit Keimen zu schützen. Als Konservierungsstoffe eignen sich die dem Stand der Technik bekannten, insbesondere Cetylpyridiniumchlorid, Benzalkoniumchlorid oder Benzoesäure bzw. Benzoate wie Natriumbenzoat in der aus dem Stand der Technik bekannten Konzentration. Die vorstehend genannten Konservierungsstoffe sind vorzugsweise in Konzentrationen von bis zu 50mg/100ml, besondersbevorzugt zwischen 5 und 20 mg/100ml enthalten.

Bevorzugte Formulierungen enthalten außer dem Lösungsmittel Wasser und der Wirkstoffkombination aus **1**, **2** und **3** nur noch Benzalkoniumchlorid und Natriumedetat. In einer anderen bevorzugten Ausführungsform wird auf Natriumedetat verzichtet.

Zur Applikation der erfindungsgemäßen treibgasfreien Inhaltionslösungen sind besonders solche Inhalatoren, die eine kleine Menge einer flüssigen Formulierung in der therapeutisch notwendigen Dosierung binnen weniger Sekunden in ein therapeutisch-inhalativ geeignetes Aerosol vernebeln können. Im Rahmen der vorliegenden Erfindung sind solche Vernebler bevorzugt, bei denen bereits eine Menge von weniger als 100 µL, bevorzugt weniger als 50 µL, besonders bevorzugt zwischen 20 und 30 µL Wirkstofflösung mit bevorzugt einem Hub zu einem Aerosol mit einer durchschnittlichen Teilchengröße von weniger als 20 µm, bevorzugt weniger als 10 µm, so vernebelt werden können, daß der inhalierbare Anteil des Aerosols bereits der therapeutisch wirksamen Menge entspricht.

Eine derartige Vorrichtung zur treibgasfreien Verabreichung einer dosierten Menge eines flüssigen Arzneimittels zur inhalativen Anwendung, wird beispielsweise in der internationalen Patentanmeldung WO 91/14468 als auch in der WO 97/12687 (dort insbesondere Figuren 6a und 6b) ausführlich beschrieben. Die dort beschriebenen Vernebler (Devices) sind auch unter der Bezeichnung Respimat® bekannt.

Dieser Vernebler (Respimat®) kann vorteilhaft zur Erzeugung der erfindungsgemäßen inhalierbaren Aerosole enthaltend die Wirkstoffkombination aus **1**, **2** und **3** eingesetzt werden. Aufgrund seiner zylinderähnlichen Form und einer handlichen Größe von weniger als 9 bis 15 cm in der Länge und 2 bis 4 cm in der Breite kann dieses Device jederzeit vom Patienten mitgeführt werden. Der Vernebler versprüht ein definiertes Volumen der Arzneimittelformulierung unter Anwendung hoher Drücke durch kleine Düsen, so daß inhalierbare Aerosole entstehen.

Im wesentlichen besteht der bevorzugte Zerstäuber aus einem Gehäuseoberteil, einem Pumpengehäuse, einer Düse, einem Sperrspannwerk, einem Federgehäuse, einer Feder und einem Vorratsbehälter, gekennzeichnet durch
- ein Pumpengehäuse, das im Gehäuseoberteil befestigt ist, und das an seinem einen Ende einen Düsenkörper mit der Düse bzw. Düsenanordnung trägt,
- einen Hohlkolben mit Ventilkörper,
- einen Abtriebsflansch, in dem der Hohlkolben befestigt ist, und der sich im Gehäuseoberteil befindet,
- ein Sperrspannwerk, das sich im Gehäuseoberteil befindet,
- ein Federgehäuse mit der darin befindlichen Feder, das am Gehäuseoberteil mittels eines Drehlagers drehbar gelagert ist,
- ein Gehäuseunterteil, das auf das Federgehäuse in axialer Richtung aufgesteckt ist.

Der Hohlkolben mit Ventilkörper entspricht einer in der WO 97/12687 offenbarten Vorrichtung. Er ragt teilweise in den Zylinder des Pumpengehäuses hinein und ist im Zylinder axial verschiebbar angeordnet. Insbesondere wird auf die Figuren 1-4 - insbesondere Figur 3 - und die dazugehörigen Beschreibungsteile Bezug genommen. Der Hohlkolben mit Ventilkörper übt auf seiner Hochdruckseite zum Zeitpunkt des Auslösens der Feder einen Druck von 5 bis 60 Mpa (etwa 50 bis 600 bar), bevorzugt 10 bis 60 Mpa (etwa 100 bis 600 bar) auf das Fluid, die abgemessene Wirkstofflösung aus. Dabei werden Volumina von 10 bis 50 Mikroliter bevorzugt, besonders bevorzugt sind Volumina von 10 bis 20 Mikroliter, ganz besonders bevorzugt ist ein Volumen von 15 Mikroliter pro Hub.

Der Ventilkörper ist bevorzugt an dem Ende des Hohlkolbens angebracht, das dem Düsenkörper zugewandt ist.

Die Düse im Düsenkörper ist bevorzugt mikrostrukturiert, d.h. durch Mikrotechnik hergestellt. Mikrostrukturierte Düsenkörper sind beispielsweise in der WO-94/07607 offenbart; auf diese Schrift wird hiermit inhaltlich Bezug genommen, insbesondere auf die dort offenbarte Figur 1 und deren Beschreibung.
Der Düsenkörper besteht z.B. aus zwei fest miteinander verbundenen Platten aus Glas und/oder Silizium, von denen wenigstens eine Platte einen oder mehrere mikrostrukturierte Kanäle aufweist, die die Düseneinlaßseite mit der Düsenauslaßseite verbinden. Auf der Düsenauslaßseite ist mindestens eine runde oder nicht-runde Öffnung von 2 bis 10 Mikrometer Tiefe und 5 bis 15 Mikrometern Breite, wobei die Tiefe bevorzugt bei 4, 5 bis 6,5 Mikrometern und die Länge bei 7 bis 9 Mikrometern beträgt.
Im Fall von mehreren Düsenöffnungen, bevorzugt sind zwei, können die Strahlrichtungen der Düsen im Düsenkörper parallel zueinander verlaufen oder sie sind in Richtung Düsenöffnung gegeneinander geneigt. Bei einem Düsenkörper mit mindestens zwei Düsenöffnungen auf der Auslaßseite können die Strahlrichtungen mit einem Winkel von 20 Grad bis 160 Grad gegeneinander geneigt sein, bevorzugt wird ein Winkel von 60 bis 150 Grad, insbesondere bevorzugt 80 bis 100°.
Die Düsenöffnungen sind bevorzugt in einer Entfernung von 10 bis 200 Mikrometern angeordnet, stärker bevorzugt in einer Entfernung von 10 bis 100 Mikrometer, besonders bevorzugt 30 bis 70 Mikrometer. Am stärksten bevorzugt sind 50 Mikrometer.
Die Strahlrichtungen treffen sich dementsprechend in der Umgebung der Düsenöffnungen.

Die flüssige Arzneimittelzubereitung trifft mit einem Eingangsdruck von bis zu 600 bar, bevorzugt 200 bis 300 bar auf den Düsenkörper und wird über die Düsenöffnungen in ein inhalierbares Aerosol zerstäubt. Die bevorzugten Teilchen- bzw. Tröpfchengrößen des Aerosols liegen bei bis zu 20 Mikrometern, bevorzugt 3 bis 10 Mikrometern.

Das Sperrspannwerk enthält eine Feder, bevorzugt eine zylindrische schraubenförmige Druckfeder, als Speicher für die mechanische Energie. Die Feder wirkt auf den Abtriebsflansch als Sprungstück, dessen Bewegung durch die Position eines Sperrglieds bestimmt wird. Der Weg des Abtriebsflansches wird durch einen oberen und einen unteren Anschlag präzise begrenzt. Die Feder wird bevorzugt über ein kraftübersetzendes Getriebe, z.B. ein Schraubschubgetriebe, durch ein äußeres Drehmoment gespannt, das beim Drehen des Gehäuseoberteils gegen das Federgehäuse im Gehäuseunterteil erzeugt wird. In diesem Fall enthalten das Gehäuseoberteil und der Abtriebsflansch ein ein- oder mehrgängiges Keilgetriebe.

Das Sperrglied mit einrückenden Sperrflächen ist ringförmig um den Abtriebsflansch angeordnet. Es besteht z.B. aus einem in sich radial elastisch verformbaren Ring aus Kunststoff oder aus Metall. Der Ring ist in einer Ebene senkrecht zur Zerstäuberachse angeordnet. Nach dem Spannen der Feder schieben sich die Sperrflächen des Sperrgliedes in den Weg des Abtriebsflansches und verhindern das Entspannen der Feder. Das Sprerrglied wird mittels einer Taste ausgelöst. Die Auslösetaste ist mit dem Sperrglied verbunden oder gekoppelt. Zum Auslösen des Sperrspannwerkes wird die Auslösetaste parallel zur Ringebene, und zwar bevorzugt in den Zerstäuber hinein, verschoben; dabei wird der verformbare Ring in der Ringebene verformt. Konstruktive Details des Sperrspannwerkes sind in der WO 97/20590 beschrieben.

Das Gehäuseunterteil wird in axialer Richtung über das Federgehäuse geschoben und verdeckt die Lagerung, den Antrieb der Spindel und den Vorratsbehälter für das Fluid.

Beim Betätigen des Zerstäubers wird das Gehäuseobereil gegen das Gehäuseunterteil gedreht, wobei das Gehäuseunterteil das Federgehäuse mitnimmt. Dabei wird die Feder über das Schraubschubgetriebe zusammengedrückt und gespannt, und das Sperrwerk rastet selbsttätig ein. Der Drehwinkel ist bevorzugt ein ganzzahliger Bruchteil von 360 Grad, z.B. 180 Grad. Gleichzeitig mit dem Spannen der Feder wird das Abtriebsteil im Gehäuseoberteil um einen vorgegebenen Weg verschoben, der Hohlkolben wird innerhalb des Zylinders im Pumpengehäuse zurückgezogen, wodurch eine Teilmenge des Fluids aus dem Vorratsbehälter in den Hochdruckraum vor der Düse eingesaugt wird.

In den Zerstäuber können gegebenenfalls nacheinander mehrere das zu zerstäubende Fluid enthaltende austauschbare Vorratsbehälter eingeschoben und benutzt werden. Der Vorratsbehälter enthält die erfindungsgemäße wässerige Aerosolzubereitung.

Der Zerstäubungsvorgang wird durch leichtes Eindrücken der Auslösetaste eingeleitet. Dabei gibt das Sperrwerk den Weg für das Abtriebsteil frei. Die gespannte Feder schiebt den Kolben in den Zylinder des Pumpengehäuses hinein. Das Fluid tritt aus der Düse des Zerstäubers in zerstäubter Form aus.

Weitere konstruktive Details sind in den PCT-Anmeldungen WO 97/12683 und WO 97/20590 offenbart, auf die hiermit inhaltlich Bezug genommen wird.

Die Bauteile des Zerstäubers (Verneblers) sind aus einem der Funktion entsprechend geeignetem Material. Das Gehäuse des Zerstäubers und -- so weit es die Funktion erlaubt - auch andere Teile sind bevorzugt aus Kunststoff, z.B. im Spritzgießverfahren, hergestellt. Für medizinische Zwecke werden physiologisch unbedenkliche Materialien verwendet.

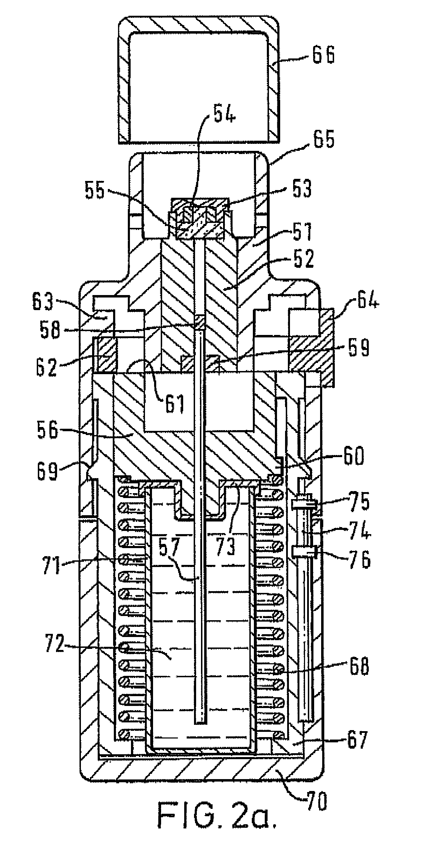

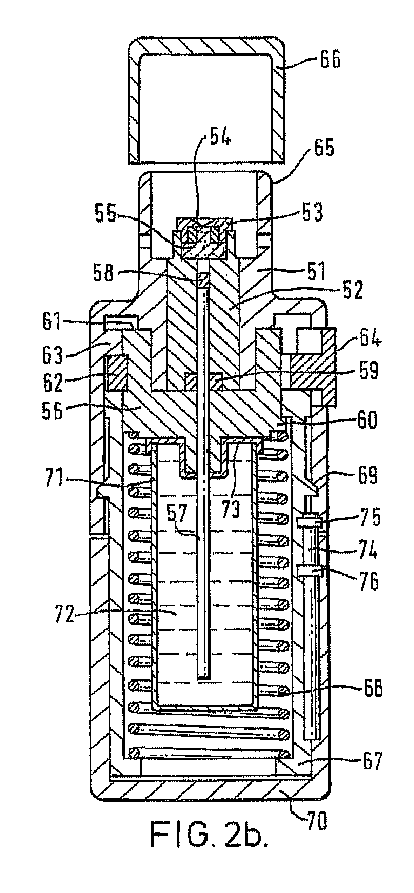

In den dieser Patentanmeldung beigefügten Figuren 2a/b, die identisch sind mit den Figuren 6 a/b der WO 97/12687, ist der Vernebler (Respimat®) beschrieben, mit dem die erfindungsgemäßen wäßrigen Aerosolzubereitungen vorteilhaft inhaliert werden können.

Figur 2a zeigt einen Längsschnitt durch den Zerstäuber bei gespannter Feder, Figur 2b zeigt einen Längsschnitt durch den Zerstäuber bei entspannter Feder.

Das Gehäuseoberteil (51) enthält das Pumpengehäuse (52), an dessen Ende der Halter (53) für die Zerstäuberdüse angebracht ist. In dem Halter befindet sich der Düsenkörper (54) und ein Filter (55). Der im Abtriebsflansch (56) des Sperrspannwerkes befestigte Hohlkolben (57) ragt teilweise in den Zylinder des Pumpengehäuses hinein. An seinem Ende trägt der Hohlkolben den Ventilkörper (58). Der Hohlkolben ist mittels der Dichtung (59) abgedichtet. Innerhalb des Gehäuseoberteils befindet sich der Anschlag (60), an dem der Abtriebsflansch bei entspannter Feder anliegt. Am Abtriebsflansch befindet sich der Anschlag (61), an dem der Abtriebsflansch bei gespannter Feder anliegt. Nach dem Spannen der Feder schiebt sich das Sperrglied (62) zwischen den Anschlag (61) und eine Abstützung (63) im Gehäuseoberteil. Die Auslösetaste (64) steht mit dem Sperrglied in Verbindung. Das Gehäuseobereil endet im Mundstück (65) und ist mit der aufsteckbaren Schutzkappe (66) verschlossen.

Das Federgehäuse (67) mit Druckfeder (68) ist mittels der Schnappnasen (69) und Drehlager am Gehäuseoberteil drehbar gelagert. Über das Federgehäuse ist das Gehäuseunterteil (70) geschoben. Innerhalb des Federgehäuses befindet sich der austauschbare Vorratsbehälter (71) für das zu zerstäubende Fluid (72). Der Vorratsbehälter ist mit dem Stopfen (73) verschlossen, durch den der Hohlkolben in den Vorratsbehälter hineinragt und mit seinem Ende in das Fluid (Vorrat an Wirkstofflösung) eintaucht.

In der Mantelfläche des Federgehäuses ist die Spindel (74) für das mechanische Zählwerk angebracht. An dem Ende der Spindel, das dem Gehäuseoberteil zugewandt ist, befindet das Antriebsritzel (75). Auf der Spindel sitzt der Reiter (76).

Der oben beschriebene Vernebler ist geeignet, die erfindungsgemäßen Aerosolzubereitungen zu einem für die Inhalation geeignetem Aerosol zu vernebeln.

Wird die erfindungsgemäße Formulierung mittels der vorstehend beschriebenen Technik (Respimat®) vernebelt, sollte die ausgebrachte Masse bei wenigstens 97%, bevorzugt wenigstens 98% aller Betätigungen des Inhalators (Hube) einer definierten Menge mit einem Toleranzbereichs von maximal 25%, bevorzugt 20% dieser Menge entsprechen. Bevorzugt werden pro Hub zwischen 5 und 30 mg Formulierung als definierte Masse ausgebracht, besonders bevorzugt zwischen 5 und 20 mg.

Die erfindungsgemäße Formulierung kann jedoch auch mittels anderer als der vorstehend beschriebenen Inahalatoren, beispielsweise Jet-Stream-Inhalatoren, vernebelt werden.

Dementsprechend betrifft ein weiterer Aspekt der vorliegenden Erfindung Arzneimittel in Form von wie vorstehend beschriebenen treibgasfreien Inhaltionslösungen oder Suspensionen in Verbindung mit einer zur Verabreichung dieser Formulierungen geeigneten Vorrichtung, bevorzugt in Verbindung mit dem Respimat®. Bevorzugt zielt die vorliegende Erfindung auf treibgasfreie Inhaltionslösungen oder Suspensionen gekennzeichnet durch die erfindungsgemäßen Wirkstoffkombination aus **1, 2** und **3** in Verbindung mit der unter der Bezeichnung Respimat® bekannten Vorrichtung. Ferner betrifft die vorliegende Erfindung vorstehend genannte Vorrichtungen zur Inhalation, bevorzugt den Respimat®, dadurch gekennzeichnet, daß sie vorstehend beschriebene erfindungsgemäße treibgasfreie Inhaltionslösungen oder Suspensionen enthalten.

Die erfindungsgemäßen treibgasfreien Inhalationslösungen oder Suspensionen können neben den vorstehend, zur Applikation im Respimat vorgesehenen Lösungen und Suspensionen auch als Konzentrate oder sterile gebrauchsfertige Inhalationslösungen bzw. -suspensionen vorliegen. Aus den Konzentraten lassen sich beispielsweise durch Zugabe von isotonischen Kochsalzlösungen gebrauchsfertige Formulierungen generieren. Sterile gebrauchsfertige Formulierungen können mittels energiebetriebener Stand- oder transportabler Vernebler, die inhalierbare Aerosole mittels Ultraschall oder Druckluft nach dem Venturiprinzip oder anderen Prinzipien erzeugen, appliziert werden.

Dementsprechend betrifft ein weiterer Aspekt der vorliegenden Erfindung Arzneimittel in Form von wie vorstehend beschriebenen treibgasfreien Inhaltionslösungen oder Suspensionen, die als Konzentrate oder sterile gebrauchsfertige Formulierungen vorliegen, in Verbindung mit einer zur Verabreichung dieser Lösungen geeigneten Vorrichtung, dadurch gekennzeichnet, daß es sich bei dieser Vorrichtung um einen energiebetriebenen Stand- oder transportablen Vernebler handelt, der inhalierbare Aerosole mittels Ultraschall oder Druckluft nach dem Venturiprinzip oder anderen Prinzipien erzeugt.

Die folgenden Beispiele dienen einer weitergehenden Erläuterung der vorliegenden Erfindung, ohne den Umfang der Erfindung allerdings auf die nachfolgenden beispielhaften Ausführungsformen zu beschränken.

### Formullerungsbeispiele

### A) Inhaltionspulver:

1)

| **Bestandteile** | **µg pro Kapsel** |
|---|---|
| **1'**-Bromid | 100 |
| Budesonid | 200 |
| Salmeterolxinafoat | 55,9 |
| Lactose | 4644,1 |
| **Summe** | 5000 |

2)

| **Bestandteile** | **µg pro Kapsel** |
|---|---|
| **1'**-Bromid | 75 |
| Fluticasone-propionat | 125 |
| Salmeterol-4-phenylcinnamat | 50 |
| Lactose | 4650 |
| **Summe** | 5000 |

3)

| **Bestandteile** | **µg pro Kapsel** |
|---|---|
| **1'**-Bromid | 75 |
| Mometasone-furoat | 250 |
| Formoterolfumaratdihydrat | 12 |
| Lactose | 4663 |
| **Summe** | 5000 |

4)

| **Bestandteile** | **µg pro Kapsel** |
|---|---|
| **1**'-Bromid | 100 |
| Fluticasone-propionat | 250 |
| Formoterolfumaratdihydrat | 12 |
| Lactose | 4638 |
| **Summe** | 5000 |

5)

| **Bestandteile** | **µg pro Kapsel** |
|---|---|
| **1'**-Bromid | 200 |
| Formoterolfumaratdihydrat | 12 |
| Fluticasone-propionat | 250 |
| Lactose | 24538 |
| **Summe** | 25000 |

6)

| **Bestandteile** | **µg pro Kapsel** |
|---|---|
| **1'**-Bromid | 75 |
| Fluticasone-propionat | 125 |
| Salmeterol-diflunisal | 50 |
| Lactose | 24750 |
| **Summe** | 25000 |

### B) Treibgashaltige Inhaltionsaerosole:

1) Suspensionsaerosol:

| **Bestandteile** | **Gew-%** |
|---|---|
| **1'**-Bromid | 0,035 |
| Budesonid | 0,4 |
| Formoterolfumaratdihydrat | 0,066 |
| Sojalecithin | 0,2 |
| TG 134a : TG227 = 2:3 | ad 100 |

2) Suspensionsaerosol:

| **Bestandteile** | **Gew%** |
|---|---|
| **1'**-Bromid | 0,039 |
| Fluticasone-propionat | 0,3 |
| Salmeterolxinafoat | 0,033 |
| Isopropylmyristat | 0,1 |
| TG 227 | ad 100 |

3) Suspensionsaerosol:

| **Bestandteile** | **Gew-%** |
|---|---|
| **1'**-Bromid | 0,039 |
| Mometasone-furoat | 0,6 |
| Salmeterol x diflunisal | 0,066 |
| Isopropylmyristat | 0,1 |
| TG 227 | ad 100 |

4) Suspensionsaerosol:

| **Bestandteile** | **Gew-%** |
|---|---|
| **1'**-Bromid | 0,035 |
| Fluticasone-propionat | 0,3 |
| Salmeterol-4-phenylcinnamat | 0,066 |
| Sojalecithin | 0,2 |
| TG 11 : TG12 = 2:3 | ad 100 |

5) Suspensionsaerosol:

| **Bestandteile** | **Gew-%** |
|---|---|
| **1'**-Bromid | 0,039 |
| Salmeterolxinafoat | 0,033 |
| Budesonid | 0,4 |
| Ethanol, absolut | 0,5 |
| Isopropylmyristat | 0,1 |
| TG 227 | ad 100 |

## Patentansprüche

1. Arzneimittel für die Inhalation **gekennzeichnet durch** einen Gehalt an wenigstens einem Anticholinergikum der Formel **1**, 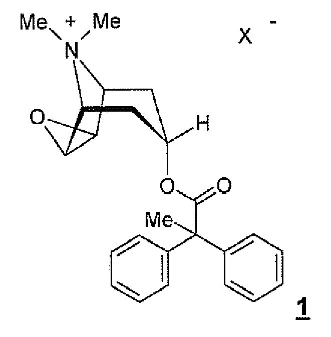 worin
X - ein einfach negativ geladenes Anion, vorzugsweise ein Anion ausgewählt aus der Gruppe bestehend aus Chlorid, Bromid, Iodid, Sulfat, Phosphat, Methansulfonat, Nitrat, Maleat, Acetat, Citrat, Fumarat, Tartrat, Oxalat, Succinat, Benzoat und p-Toluolsulfonat bedeutet,
wenigstens einem Corticosteroid (2) sowie wenigstens einem Betamimetikum (3), gegebenenfalls in Form ihrer Enantiomere, Gemische der Enantiomere oder in Form der Racemate, gegebenenfalls in Form der Solvate oder Hydrate sowie gegebenenfalls gemeinsam mit einem physiologisch unbedenklichen Hilfsstoff.

2. Arzneimittel nach Anspruch 1, **dadurch gekennzeichnet, daß** die Wirkstoffe **1**, **2** und **3** entweder gemeinsam in einer einzigen Darreichungsform oder in zwei oder drei getrennten Darreichungsformen enthalten sind.

3. Arzneimittel nach einem der Ansprüche 1 oder 2, **dadurch gekennzeichnet, daß 1** ausgewählt ist aus der Gruppe der Salze, in denen X - für ein einfach negativ geladenes Anion ausgewählt aus der Gruppe bestehend aus Chlorid, Bromid, 4-Toluolsulfonat und Methansulfonat, bevorzugt Bromid steht.

4. Arzneimittel nach einem der Ansprüche 1 bis 3, **dadurch gekennzeichnet, daß 2** ausgewählt ist aus der Gruppe bestehend aus Flunisolide, Beclomethasone, Triamcinolone, Budesonid, Fluticasone, Mometasone, Ciclesonide, Rofleponide, GW 215864, KSR 592, ST-126 und Dexametasone.

5. Arzneimittel nach Anspruch 4, **dadurch gekennzeichnet, daß 2** ausgewählt ist aus der Gruppe bestehend aus Flunisolide, Beclomethasone, Triamcinolone, Budesonid, Fluticasone, Mometasone, Ciclesonide und Dexametasone.

6. Arzneimittel nach einem der Ansprüche 1 bis 5, **dadurch gekennzeichnet, daß 3** ausgewählt ist aus der Gruppe bestehend aus Bambuterol, Bitolterol, Carbuterol, Clenbuterol, Fenoterol, Formoterol, Hexoprenalin, Ibuterol, Pirbuterol, Procaterol, Reproterol, Salmeterol, Sulfonterol, Terbutalin, Tolubuterol, 4-Hydroxy-7-[2-{[2-{[3-(2-phenylethoxy)propyl]sulfonyl}ethyl]-amino}ethyl]-2(3H)-benzothiazolon,
1-(2-Fluoro-4-hydroxyphenyl)-2-[4-(1-benzimidazolyl)-2-methyl-2-butylamino]ethanol, 1-[3-(4-Methoxybenzyl-amino)-4-hydroxyphenyl]-2-[4-(1-benzimidazolyl)-2-methyl-2-butylamino]ethanol,
1-[2H-5-Hydroxy-3-oxo-4H-1,4-benzoxazin-8-yl]-2-[3-(4-N,N-dimethylaminophenyl)-2-methyl-2-propylamino]ethanol,
1-[2H-5-Hydroxy-3-oxo-4H-1,4-benzoxazin-8-yl]-2-[3-(4-methoxyphenyl)-2-methyl-2-propylamino]ethanol,
1-[2H-5-Hydroxy-3-oxo-4H-1,4-benzoxazin-8-yl]-2-[3-(4-n-butyloxyphenyl)-2-methyl-2-propylamino]ethanol ,
1-[2H-5-Hydroxy-3-oxo-4H-1,4-benzoxazin-8-yl]-2-{4-[3-(4-methoxyphenyl)-1,2,4-triazol-3-yl]-2-methyl-2-butylamino]ethanol,
5-Hydroxy-8-(1-hydroxy-2-isopropylaminobutyl)-2H-1,4-benzoxazin-3-(4H)-on,
1-(4-Amino-3-chloro-5-trifluormethylphenyl)-2-tert-butylamino)ethanol und
1-(4-Ethoxycarbonylamino-3-cyano-5-fluorophenyl)-2-(tert.-butylamino)ethanol.

7. Arzneimittel nach Anspruch 6, **dadurch gekennzeichnet, daß 3** ausgewählt ist aus der Gruppe bestehend aus Formoterol, Salmeterol,
4-Hydroxy-7-[2-{[2-{[3-(2-pheny)ethoxy)propyl]sulfonyl}ethyl]-amino}ethyl]-2(3H)-benzothiazolon,
1-(2-Fluoro-4-hydroxyphenyl)-2-[4-(1-benzimidazolyl)-2-methyl-2-butylamino]ethanol, 1-[3-(4-Methoxybenzyl-amino)-4-hydroxyphenyl]-2-[4-(1-benzimidazolyl)-2-methyl-2-butylamino]ethanol,
1-[2H-5-Hydroxy-3-oxo-4H-1,4-benzoxazin-8-yl]-2-[3-(4-N,N-dimethylaminophenyl)-2-methyl-2-propylamino]ethanol,
1-[2H-5-Hydroxy-3-oxo-4H-1,4-benzoxazin-8-yl]-2-[3-(4-methoxyphenyl)-2-methyl-2-propylamino]ethanol,
1-[2H-5-Hydroxy-3-oxo-4H-1,4-benzoxazin-8-yl]-2-[3-(4-n-butyloxyphenyl)-2-methyl-2-propylamina]ethanol und
1-[2H-Hydroxy-3-oxo-4H-1 ,4-benzoxazin-8-yl]-2-{4-[3-(4-methoxyphenyl)-1,2,4-triazol-3-yl]-2-methyl-2-butylamino}ethanol.

8. Arneimittel nach einem der Ansprüche 1 bis 7, **dadurch gekennzeichnet, daß** die Gewichtsverhältnisse van **1'** zu **2** in einem Bereich von 1:250 bis 250:1, bevorzugt von 1:150 bis 150:1 liegen.

9. Arneimittel nach einem der Ansprüche 1 bis 8, **dadurch gekennzeichnet, daß** die Gewichtsverhältnisse von **1** zu **3** in einem Bereich von 1:300 bis 30:1, bevorzugt von 1:230 bis 20:1 liegen.

10. Arzneimittel nach einem der Ansprüche 1 bis 9, **dadurch gekennzeichnet, daß** eine einmaliger Applikation einer Dosierung der Wirkstoffkombination **1**, **2** und **3** von 1 bis 10000µg, bevorzugt von 10 bis 2000µg entspricht.

11. Arzneimittel nach einem der Ansprüche 1 bis 10, **dadurch gekennzeichnet, daß** es in Form einer für die Inhalation geeigneten Darreichungsform vorliegt.

12. Arzneimittel nach Anspruch 11, **dadurch gekennzeichnet, daß** es sich um eine Darreichungsform ausgewählt aus der Gruppe inhalationspulver, treibgashaltige Dosieraerosole und treibgasfreie Inhalationslösungen oder -suspensionen handelt.

13. Arzneimittel nach Anspruch 12, **dadurch gekennzeichnet, daß** es ein Inhalationspulver ist, welches **1, 2** und **3** im Gemisch mit geeigneten physiologisch unbedenkliche Hilfsstoffen ausgewählt aus der Gruppe bestehend aus Monosacchariden, Disacchariden, Oligo- und Polysacchariden, Polyalkoholen, Salzen, oder Mischungen dieser Hilfsstoffe enthält.

14. Inhalationspulver nach Anspruch 13, **dadurch gekennzeichnet, daß** der Hilfsstoff eine maximale mittlere Teilchengröße von bis zu 250µm, bevorzugt zwischen 10 und 150µm aufweist.

15. Kapseln **gekennzeichnet durch** einen Gehalt an Inhaltionspulver nach Anspruch 13 oder 14.

16. Arzneimittel nach Anspruch 12, **dadurch gekennzeichnet, daß** es ein Inhalationspulver ist, welches als Bestandteile lediglich die Wirkstoffe **1**, **2** und **3** enthält.

17. Arzneimittel nach Anspruch 12, **dadurch gekennzeichnet, daß** es sich um ein treibgashaltiges Inhalationsaerosol handelt, welches **1**, **2** und **3** in gelöster oder dispergierter Form enthält.

18. Treibgashaltiges Inhalationsaerosol nach Anspruch 17, **dadurch gekennzeichnet, daß** es als Treibgas Kohlenwasserstoffe wie n-Propan, n-Butan oder Isobutan oder Halogenkohlenwasserstoffe wie chlorierte und/oder fluorierte Derivate des Methans, Ethans, Propans, Butans, Cyclopropans oder Cyclobutans enthält.

19. Treibgashaltiges Inhaltionsaerosol nach Anspruch 18, **dadurch gekennzeichnet, daß** das Treibgas TG134a, TG227 oder ein Gemisch davon darstellt.

20. Treibgashaltiges Inhalationsaerosol nach Anspruch 17, 18 oder 19, **dadurch gekennzeichnet, daß** es gegebenenfalls einen oder mehrere weitere Bestandteile ausgewählt aus der Gruppe bestehend aus Kosolventien, Stabilisatoren, oberflächenaktive Mittel (surfactants), Antioxidantien, Schmiermittel und Mittel zur Einstellung des pH-Werts enthält.

21. Treibgashaltiges Inhalationsaerosol nach einem der Ansprüche 17 bis 20, **dadurch gekennzeichnet, daß** es bis zu 5 Gew-% an Wirkstoff **1**, **2** und/oder **3** enthalten kann.

22. Verwendung einer Kapsel gemäß Anspruch 15 in einem Inhalator, bevorzugt im Handihaler.

23. Verwendung einer Zusammensetzung nach einem der Ansprüche 1 bis 21 zur Herstellung eines Medikaments zur Behandlung von entzündlichen und/oder obstruktiven Atemwegserkrankungen.

24. Verwendung einer Zusammensetzung nach Anspruch 23 zur Herstellung eines Medikaments zur Behandlung von asthma oder COPD.
